# EUROPEAN PATENT APPLICATION

(11) **EP 1 217 366 A2**
(43) Date of publication of application: **26.06.2002**
(21) Application number: 01124514.9
(22) Date of filing: 12.10.2001
(51) Int. Cl.: G01N 27/447

(54) **Structure, method of manufacturing the same and DNA separation device using the same**

(30) Priority: 19.12.2000 JP 2000385396
(71) Applicant: MITSUBISHI DENKI KABUSHIKI KAISHA, Tokyo 100-8310 (JP)
(72) Inventor: Izuo, Shinichi, Chiyoda-ku, Tokyo 100-8310 (JP); Ohji, Hiroshi, Chiyoda-ku, Tokyo 100-8310 (JP); Tsutsumi, Kazuhiko, Chiyoda-ku, Tokyo 100-8310 (JP)
(74) Representative: Meissner, Bolte & Partner

(57) **Abstract**

The invention relates to providing a columnar structure having a uniform shape and excellent heat resistance and mechanical strength that is formed on a substrate of silicon, a method of preparing the structure, and a DNA separation device prepared by the method. The structure has, on a substrate (11) made of silicon, columns (12) the main surface of which is covered with a thermally oxidized film (16). The columns (12) are made of the thermally oxidized film (16) only or of the thermally oxidized film (16) and silicon. The thermally oxidized film formed on the columns (12) is connected to those formed on the surface or inside of the substrate (11).

## Description

### BACKGROUND OF THE INVENTION

The present invention relates to a structure having a microscopic columnar structure on a silicon substrate, the method of manufacturing the structure, and a DNA separation device using the structure.

With the recent growing importance of the DNA analyzing technology, many improvements are made to DNA separation technology as an important technique.

Known as one of such separation methods is electrophoresis. This method uses a gel or polymer as a separation carrier for electrophoresis.

Recently it is shown, for example, in PHYSICAL REVIEW LETTERS Vol. 80 pp. 1552 - 1555 (1998) that a columnar structure having microscopic gaps formed on a semiconductor substrate exhibits a DNA separation action similar to the above-mentioned gel or polymer. In this paper, it is estimated based on numerical analysis that a semiconductor substrate having columns with a pitch of 10 nm to 4 µm can separate DNAs sized between 1 bp and 50,000 bp. As mentioned above, formation of a columnar structure on a semiconductor substrate could allow electrophoresis without using a gel or polymer. When a DNA amplifying chamber, separation device, and detection unit can be integrated on a semiconductor substrate, analyzing time can be saved and a DNA analysis device can be prepared on a single semiconductor substrate, so that the manufacturing cost can be reduced.

In order to reduce resistance provided when DNAs go through the columns, it is preferable to set the height of the columnar structure to 10 µm or greater.

A method of forming a columnar structure having microscopic gaps on a semiconductor substrate is shown below.

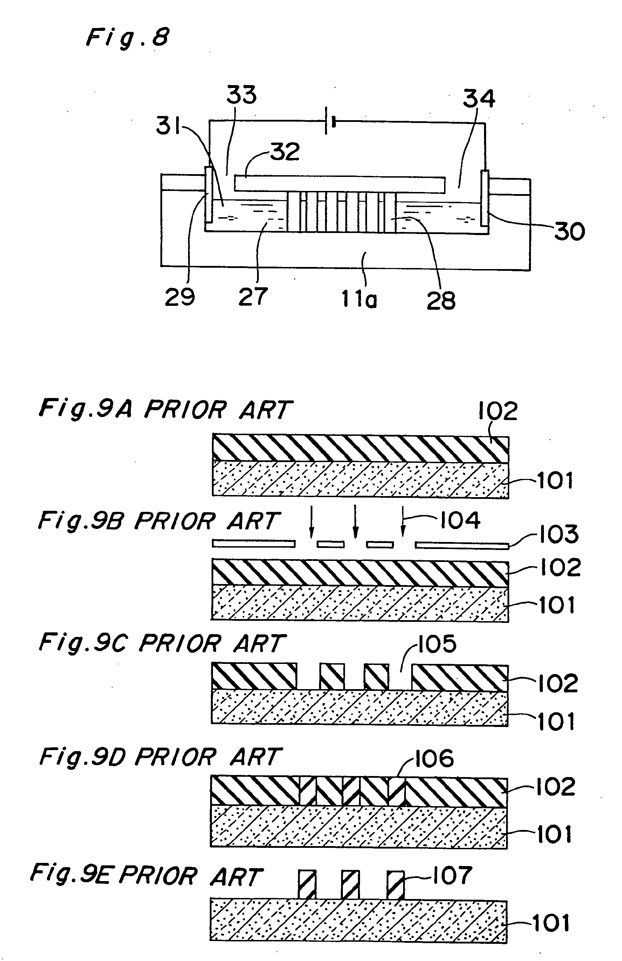

Figs. 9A to 9E illustrate a process of forming a mold on a semiconductor substrate and charging a material in the mold, which is disclosed, for example, in JP, 05-159996, A. The step proceeds from Fig. 9A to Fig. 9E. In Fig. 9A, a photoresist 102 is applied to a semiconductor substrate 101. In Fig. 9B, a mask 103 with a predetermined pattern of structure drawn therein is placed over the photoresist 102 and the photoresist 102 is irradiated with exposure light 104 through this mask 103 to prepare a pattern shown in Fig. 9C. Next, in Fig. 9D, a material 106 is charged in cavities 105 made in the photoresist and the rest of the photoresist 102 is removed. Thus, a columnar structure 107 shown in Fig. 9E can be formed. The height of the columnar structure is equal to the thickness of the photoresist film. The methods of charging materials in Fig. 9D include injection molding and electrochemical deposition.

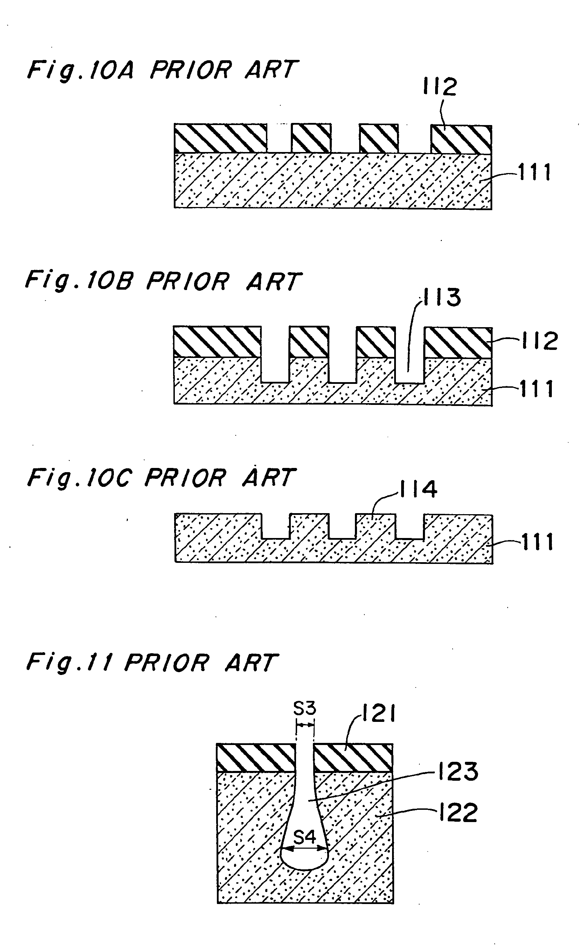

Another method is that of forming a columnar structure by etching a semiconductor substrate. Figs. 10A to 10C illustrate a process, for example, disclosed in JP, 06-333836, A. In Fig. 10A, an etching-resistant material 112 is applied to a semiconductor substrate 111 as a pattern. Next, in Fig. 10B, the areas of a semiconductor 113 that are not covered with the etching-resistant material are etched. At last, in Fig. 10C, the etching-resistant material 112 is removed and a columnar structure 114 is formed.

Those known as other methods include selectively forming columnar structures in predetermined areas, and etching by electron beams instead of a photoresist.

Another method of forming columns is that of using anodic oxidation of aluminum, for example, as disclosed in JP, 2000-31462, A. Anodic oxidation of an aluminum plate in an acid electrolyte forms a porous oxide film. An oxide film formed by anodic oxidation has a self-organizing regular microstructure. Methods of charging materials in the structure made by anodic oxidation to form microscopic columns are also known.

As mentioned above, the conventional methods of forming microscopic columns are categorized as follows:
(1) forming a structure and charging a material into the recesses of the structure to use the material as a columnar structure; and
(2) etching a semiconductor substrate to form a columnar structure.

In the above-mentioned method of forming a mold on a semiconductor substrate by a photoresist and charging a material in the mold to form a columnar structure, the height of the columnar structure depends on the thickness of the photoresist. When a photoresist has a thickness of 10 µm or greater, conventional ultra-violet rays are not sufficient for exposure, so that such a thick photoresist film is exposed to radiation light. This exposure to radiation light has disadvantages of higher cost and longer exposure time. Furthermore, there are other disadvantages: bubbles must be removed when the material is charged into the microscopic cavities after the photoresist is exposed to the light; the charged material and the substrate are not closely adhered; and the strength of the columnar structure deteriorates because heterogeneity of the substrate and structure produces a stress therebetween.

The structure made by the anodic oxidation of aluminum has a self-organizing microstructure with a thickness of 10 µm or greater. With the method of charging a material in the microstructure made by the anodic oxidation of aluminum, a microscopic columnar structure can easily be formed. However, this method has also disadvantages: the charged material and substrate are not closely adhered; and the strength of the columnar structure deteriorates because of a stress produced between the structure and substrate.

In the method of using said photoresist as an etching-resistant material and etching the areas of a semiconductor that are not covered with the photoresist to form a columnar structure, with an opening width of 1 µm or smaller, the etched width S4 is larger than the resist pattern width S3 as shown in Fig. 11. In other words, an undercut tends to develop and accurately etching a dimension of 1 µm or smaller is difficult. In addition, with an etching depth of 40 µm or greater, a photoresist with a thickness of 1 µm or greater is required. Therefore, when a pattern having a width of 1 µm or smaller is drawn in a photoresist 1 µm thick, radiation light must be used for exposure instead of conventional ultra-violet rays, which makes the manufacturing cost higher.

In the method of etching by electron beams instead of a photoresist, etching is performed sequentially and takes longer time. In addition, it is difficult to form a structure with vertical walls having a height of 10 µm or greater.

Self-organizing microscopic holes can also be formed by the method of electrochemically etching silicon. However, the method of charging a material in microscopic holes to form a structure has problems similar to those of the method of anodic oxidation of aluminum. That is, the charged material and the substrate are not closely adhered to each other and the strength of the columnar structure deteriorates because of a stress produced between the structure and substrate.

### SUMMARY OF THE INVENTION

The present invention addresses these problems. Therefore, it is an object of the present invention to provide a structure that has, on a semiconductor substrate, a columnar structure with a uniform shape, and sufficient heat resistance and mechanical strength, a method of manufacturing the structure and a DNA separation device prepared by the manufacturing method.

The present invention provides a structure for use in DNA separation, including a semiconductor substrate and a plurality of columns formed on the semiconductor substrate and at least of which surface layer is made of an oxide of the semiconductor. The structure is characterized in that passage of DNAs through the columns enables separation of the DNAs.

The present invention specifies that the height of said columns from the surface of said semiconductor substrate is between 1 µm and 1 mm.

The present invention specifies that the height of said columns is 10 µm.

The present invention specifies that part of said column is embedded in a hole provided in the surface of said semiconductor substrate.

The present invention specifies that the pitch of said adjacent columns is between 10 nm and 4 µm.

The present invention specifies that said semiconductor substrate is made of silicon and said oxide is made of a silicon oxide.

The present invention specifies that the silicon is n-type silicon.

The present invention specifies that the surface layer of said columns is a thermally oxidized layer of said semiconductor.

The present invention specifies that the interior of said column is made of one selected between said semiconductor and a hollow space.

The present invention also provides a method of manufacturing a structure for use in DNA separation including: a step of preparing a semiconductor substrate; a step of forming a mask layer with a plurality of openings on the surface of the semiconductor substrate; an etching step of immersing the semiconductor substrate in an etching liquid so as to etch the semiconductor substrate exposed in the openings and form holes; a step of thermally oxidizing the semiconductor substrate and forming a thermally oxidized film so that the film covers the surface of the holes; a step of removing the mask layer; and a step of etching the semiconductor substrate from the surface thereof so that the thermally oxidized film protrudes from the surface of the semiconductor substrate and forming columns at least of which surface is made of the thermally oxidized film.

The present invention specifies that the etching step is an electrolytic etching step in which said semiconductor substrate is immersed in a solution containing hydrofluoric acid and used as an anode for etching.

The present invention specifies that said etching step is a step of using n-type silicon as the semiconductor and performing electrolytic etching of the semiconductor substrate while irradiating the back of the semiconductor substrate with light with a wavelength of 1,100 nm or smaller.

The present invention specifies that the manufacturing method includes a step of forming microscopic asperities on the surface of said semiconductor substrate exposed in the openings prior to said etching step.

The present invention also provides a DNA separation device for use in DNA separation including: a semiconductor substrate; a recess provided in the surface of the semiconductor substrate so as to hold a liquid; a plurality of columns provided at the bottom of the recess and at least of which surface layer is made of an oxide of the semiconductor; and a pair of electrodes sandwiching the columns. The device is characterized in that voltage is applied across the electrodes so that the DNAs in the liquid held in the recess perform electrophoresis through the columns.

The present invention specifies that the height of said columns from the bottom of the recess is between 1 µm and 1 mm.

The present invention specifies that the height of said columns is 10 µm.

The present invention specifies that the pitch of said adjacent columns is between 10 nm and 4 µm.

The present invention specifies that said semiconductor substrate is made of silicon and said oxide is a silicon oxide.

The present invention specifies that the surface layer of said columns is a thermally oxidized layer of said semiconductor.

The present invention specifies that the interior of said column is made of one selected between said semiconductor and a hollow space.

As mentioned above, the structure with a columnar structure in accordance with the present invention has a columnar structure on the surface or inside of a semiconductor substrate. The columnar structure is 10 µm or greater in height, connected to said semiconductor substrate and at least surface layer thereof is made of an oxide of the semiconductor. The minimum gap of said adjacent columns is between 10 nm and 4 µm. Said semiconductor is made of silicon or n-type silicon and the surface layer of the columnar structure is a thermally oxidized layer. The interior of the column is made of the semiconductor or a hollow space. With such a constitution, a structure having an excellent mechanical and thermal strength can be provided.

The method of manufacturing the structure having a columnar structure in accordance with the present invention includes the following steps: using a semiconductor substrate as an anode and performing electrolytic etching in a solution containing hydrofluoric acid to form holes; thermally oxidizing the semiconductor substrate and forming thermally oxidized film around the holes; and removing a part of the semiconductor that is not oxidized and in the vicinity of the oxidized film. Consequently, the structure with a columnar structure is formed so that at least the surface layer of the columnar structure is the thermally oxidized film. In addition, said step of forming holes includes a step of forming microscopic asperities on the semiconductor substrate prior to the electrolytic etching. Moreover, the semiconductor is n-type silicon and the electrolytic etching step for forming holes includes a step of irradiating the back of said semiconductor substrate to be etched with light with a wavelength of 1,100 nm or smaller. With such steps, formation of a structure having an inter-column gap smaller than that can be attained with conventional photolithography or etching techniques. In addition, the use of silicon as a semiconductor allows accurate etching and thermal oxidation and thus accurate structure can be prepared.

In the DNA separation device in accordance with the present invention, a recessed channel is formed around any type of the above-mentioned columnar structures provided on the structure, and plural kinds of DNAs in the channel are separated with respect to the size by the application of electric field or pressure across the channel. With such a constitution, an element having DNA amplification, separation and detection devices can be formed on a semiconductor substrate, which can shorten analysis time, downsize the analysis device and reduce cost.

### BRIEF DESCRIPTION OF THE DRAWINGS

Fig. 1 shows a constitution (photograph: perspective view) of a columnar structure in accordance with Embodiment 1 of the present invention;
Fig. 2 shows a constitution (photograph: top view) of the columnar structure in accordance with Embodiment 1 of the present invention;
Fig. 3A is a schematic plan view of a structure in accordance with Embodiment 1 of the present invention;
Fig. 3B is a cross sectional view of the structure in accordance with Embodiment 1 of the present invention;
Figs. 4A to 4F are cross sectional views illustrating a manufacturing process of the structure in accordance with Embodiment 1 of the present invention;
Figs. 4C' to 4F' are plan views seen from the top, illustrating the manufacturing process of the structure in accordance with Embodiment 1 of the present invention;
Fig. 5 is a schematic drawing of a light-irradiating electrolytic etching apparatus for use in the manufacturing process of the structure in accordance with Embodiment 1 of the present invention;
Figs. 6A to 6E are cross- sectional views illustrating a manufacturing process of a structure in accordance with Embodiment 1 of the present invention;
Figs. 7A to 7D are cross sectional views illustrating a manufacturing process of a structure in accordance with Embodiment 2 of the present invention;

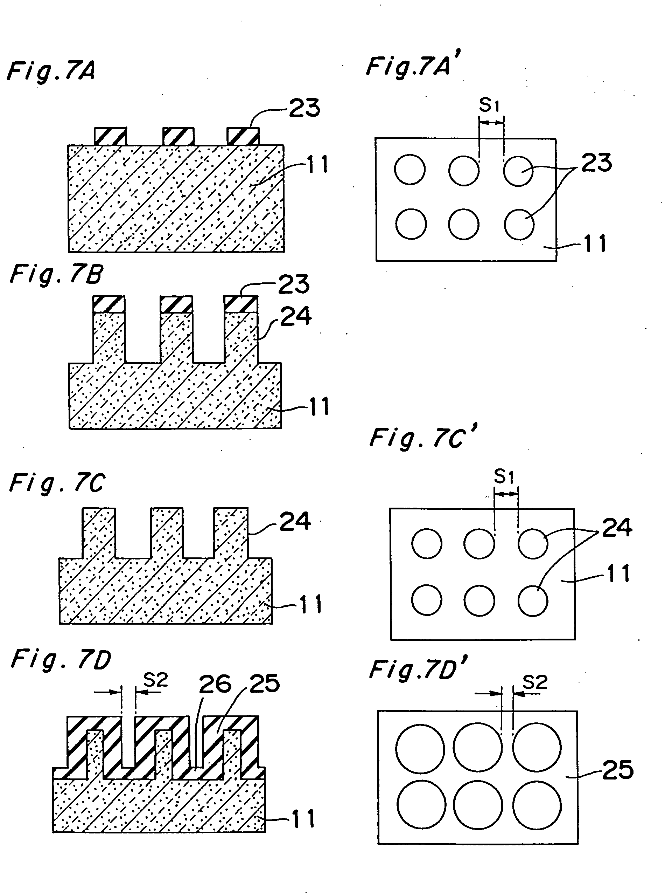

Figs. 7A', 7C' and 7D' are plan views illustrating the manufacturing process of the structure in accordance with Embodiment 2 of the present invention;
Fig. 8 is a cross sectional view illustrating an electrophoresis device for DNA separation in accordance with Embodiment 3 of the present invention;
Figs. 9A to 9E are drawings for explaining a method of forming a conventional columnar structure;
Figs. 10A to 10C are drawings for explaining another method of forming a conventional columnar structure; and
Fig. 11 is a drawing for explaining problems posed when a conventional columnar structure is formed by etching of a semiconductor substrate.

### DETAILED DESCRIPTION OF THE PREFERRED EMBODIMENTS

### Embodiment 1

### Columnar structure

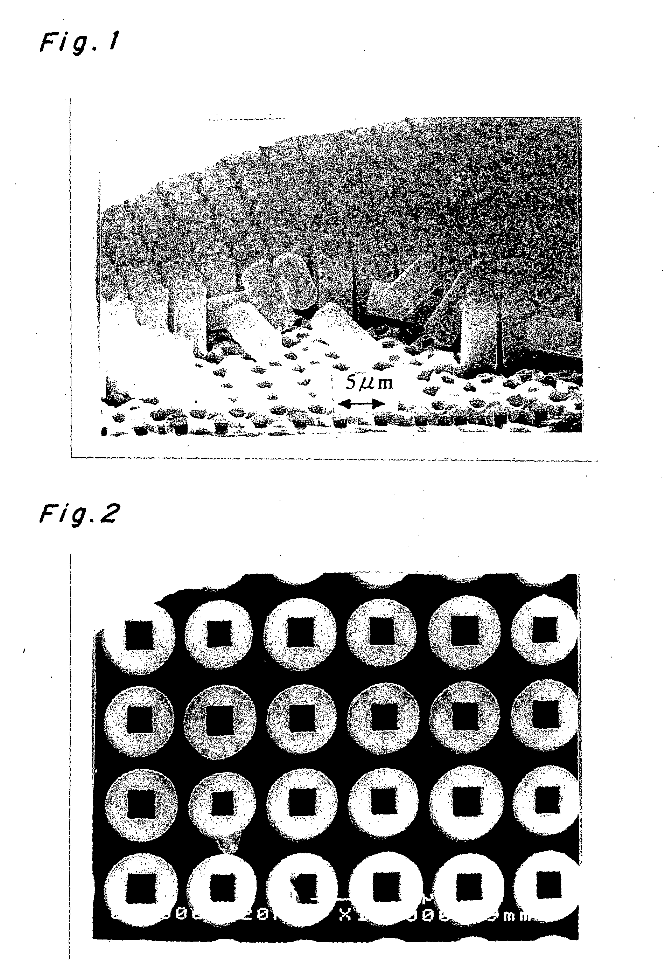

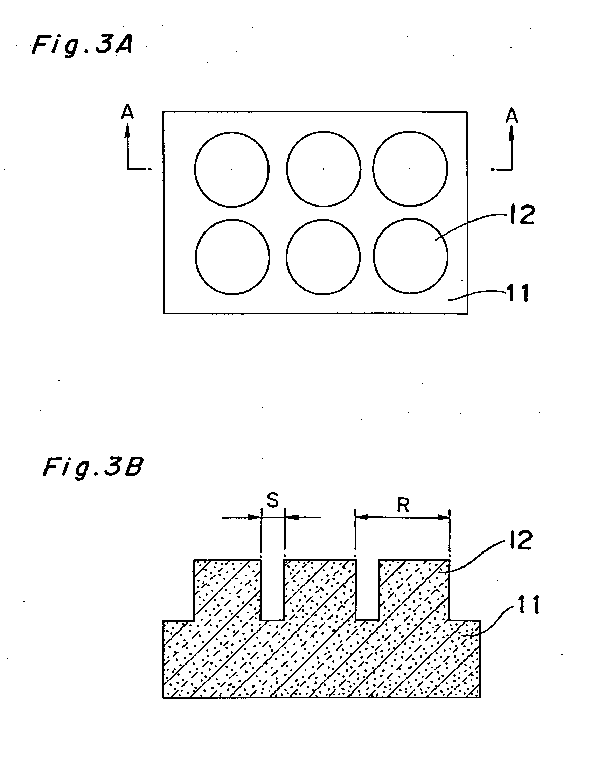

An embodiment in accordance with the present invention is hereinafter demonstrated with reference to the accompanying drawings. Figs. 1 and 2 are drawings (photographs) showing the constitution of a columnar structure in accordance with the present invention. Fig.1 is a perspective view and shows that the structure lacks a part of front columns at the top thereof. Fig. 2 shows an area of the structure having the uniformly arranged columns seen from the top thereof. White circular shapes as if extending in the drawing are columns. Fig. 3A is a schematic plan view showing the constitution of the photograph of the columnar structure shown in Fig. 2 and Fig. 3B shows a schematic sectional view taken in the direction of arrows along line A-A of Fig. 3A.

In Figs. 3A and 3B, reference numeral 12 shows a columnar structure erecting in a direction substantially perpendicular to the surface of a silicon substrate 11. The cross section of the columnar structure 12 can be circular or oval. As shown in the following process, the columnar structure 12 is made of a thermally oxidized film. The interior of the columnar structure is silicon or a hollow space and the columnar structure is not necessarily a uniform medium. As long as the pitch R of microscopic columns is between 1 µm and 20 µm, the microscopic columns need not be arranged uniformly as shown in Fig. 2. The smallest gap S between the columns is between 10 nm and 1 µm. Preferably, the height of the microscopic column is between 1 µm and 1 mm, and more preferably, is 10 µm or greater.

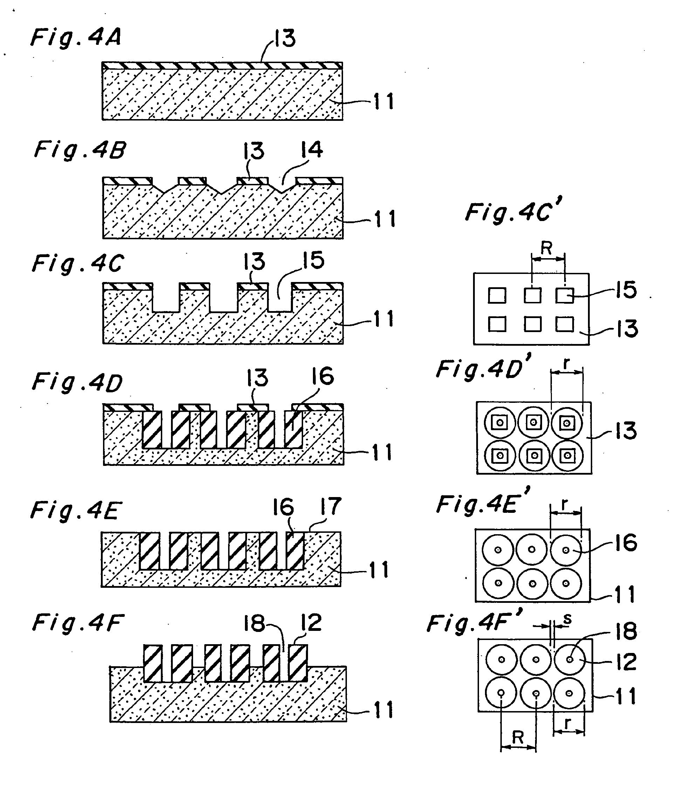

Next, a manufacturing method of said structure is described. Fig. 4 shows a process of manufacturing said microscopic columnar structure. The step proceeds from Figs. 4A to 4F. Figs. 4C', 4D', 4E' and 4F' are the drawings of Figs. 4C, 4D, 4E and 4F as seen from the top, respectively.

Firstly, as shown in Fig. 4A, a nitride film 13 is formed over the silicon substrate 11. The nitride film 13 can be formed using any method including spattering and chemical vapor deposition.

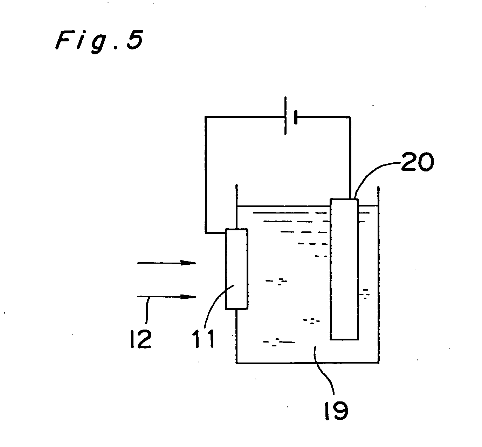

Secondly, the silicon is electrochemically etched. Fig. 5 shows a schematic drawing of an etching apparatus. The silicon substrate 11 is placed in solution 19 containing hydrofluoric acid. A predetermined voltage is applied across the anode of the silicon substrate 11 and a cathode 20 so as to pass a current therebetween. When the silicon is etched, positive holes of silicon are necessary. Therefore, with an n-type silicon substrate, the silicon substrate must be irradiated with light 21 with a wavelength of 1,100 nm or smaller.

As shown in Fig. 4B, prior to the electrochemical etching of the silicon, the nitride film 13 on the silicon substrate 11 is patterned to form microscopic cavities 14 in the exposed parts of the silicon. The patterning of nitride film can be made using ordinary photolithography technique and dry etching technique. The cavities 14 can be made using dry etching technique or using such alkali solutions as potassium hydroxide. The pitch R of the columns finally produced is equal to that of the cavities. The silicon substrate that has undergone the above-mentioned process is etched by said electrochemical etching apparatus. The etching of the silicon starts from the cavities 14, and holes 15 having vertical walls as shown in Figs. 4C and 4C' with the pitch R can be formed. In the experiment, the etching operation was performed with adjusted amount of the irradiation light 21, using an n-type silicon substrate with a resistance of 2 Ω·cm as the substrate, platinum as the cathode 20, and a solution containing 5 weight percent of hydrofluoric acid as the solution 19. When etching was performed for 30 minutes with the current value set to approx. 10mA/cm² and the potential difference from the cathode set to 1 V, a columnar structure having a diameter of 1 µm, a pitch of holes of 2 µm and a depth of holes of 40 µm was accomplished.

Thirdly, as shown in Fig. 4D, after being etched to a predetermined depth, the silicon substrate is thermally oxidized. The thermal oxidation is performed in an atmosphere of dry or wet oxygen at a temperature between 900 °C and 1,400 °C. In this thermal oxidation process, a thermally oxidized film 16 is formed around the holes 15. During the process of thermally oxidizing the silicon to form a silicon oxide, volume expansion occurs. Thus, the resultant diameter of the hole is r as shown in Fig. 4D'. The thickness and shape of the thermally oxidized film can be adjusted by the thermal oxidation time, temperature and composition of the gaseous atmosphere. When said sample is thermally oxidized in an atmosphere containing water vapor at a temperature of 1000 °C for 100 minutes, an oxidized film approx. 800 nm thick is formed.

Fourthly, as shown in Fig. 4E, the surface layer of the nitride film 13 is selectively removed. The use of phosphoric acid solution can etch only the nitride film without damaging the oxide film, and a silicon surface 17 appears. The removal of the nitride film can be accomplished not only by the phosphoric acid solution but also by ion beam etching or mechanical polishing.

Lastly, as shown in Fig. 4F, the silicon is etched. The use of such alkali solutions as tetra methyl ammonium hydroxide solution or dry etching, for example, in XeF₂ gas can etch only the silicon without damaging the oxidized film 16. When the etching is performed to a predetermined depth, the columnar structure 12 erecting on the silicon substrate 11 is formed.

The columnar structure in accordance with the present invention is made of a thermally oxidized film with a center hollow space 18. The thermally oxidized film is connected to that formed inside of the substrate. In some cases where the diameter of the holes formed by the electrolytic etching and the thickness of the thermally oxidized film are adjusted, no hollow spaces are made. When said sample was etched in tetra methyl ammonium hydroxide solution for 10 minutes, a columnar structure with a pitch of 2 µm, an inter-column gap of 200 nm and a height of 20 µm was formed. By controlling the electrochemical etching and the thickness of the thermally oxidized film, the smallest gap can be made to 10 nm and the height can be made to approx. 100 µm.

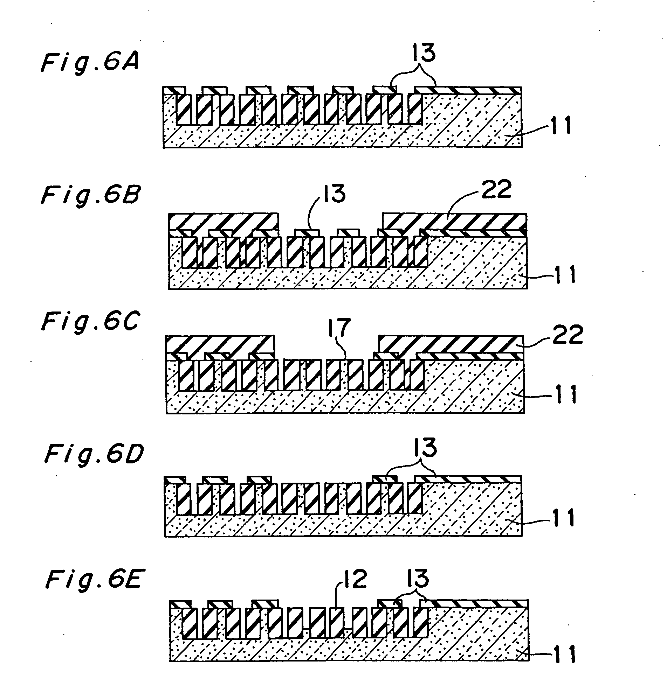

In the above embodiment, an example of forming a structure over the whole area of a silicon substrate is described. However, the structure can be prepared only in predetermined areas. Figs. 6A to 6E show the process of forming a structure only in a selected area. Fig. 6A is a cross sectional view of a sample that has been subjected to the process until the thermal oxidation shown in Fig. 4D. As shown in Fig. 6B, a photosensitive photoresist 22 is applied to the surface of the nitride film 13 to pattern the substrate. The patterning is performed so that the part of photoresist where formation of the columnar structure is desired is removed. Next, as shown in Fig. 6C, the parts of the nitride film 13 without photoresist are removed. The removing methods include ion milling by plasma of such inert gas as Ar, or reactive plasma etching in plasma with a CF₄: CHF₃: He mixing ratio of 2:1:2. Lastly, removing the photoresist 22 (as shown in Fig. 6D) and etching the silicon will form the columnar structure 12 in the desired area.

### Embodiment 2

### Columnar Structure

Another manufacturing method of a columnar structure in accordance with the preset invention is described. Figs. 7A to 7D show a process of manufacturing the columnar structure. The step proceeds from Figs. 7A to 7D. Figs.7A', 7C' and 7D' are the drawings of Figs. 7A, 7C and 7D as seen from the top, respectively.

In Fig. 7A, a photoresist 23 is applied to the silicon substrate 11 for patterning as shown in the drawing. The shape of the pattern can be an oval instead of a circle. Inter-column gap S1 in the photoresist is between 1 µm to 5 µm.

After the patterning of the photoresist in said shape, dry etching is performed as shown in Fig. 7B. At this time, employing a dry etching method with vertical anisotropy is desirable. In fact, a structure having vertical walls with an opening of 1 µm and a depth of 40 µm could be formed by the plasma etching using fluorocarbon as a reaction gas. Reference numeral 24 in the drawing shows a structure formed by the etching.

After this step, as shown in Fig. 7C, the photoresist 23 on the structure 24 is removed by dry etching or using organic solvent cleaning. Lastly, as shown in Fig. 7D, the structure 24 is thermally oxidized and a desirable columnar structure 25 is formed.

Volume expansion occurring during the process of forming a silicon oxide by the thermal oxidation of the silicon makes the inter-column gap S2 smaller than the gap S1 shown in Fig. 7C. In other words, the inter-column gap is strictly adjusted by the thermal oxidation process. According to the experiment, the smallest gap can be adjusted to 10 nm. It is proved that the inter-column gap S2 can be made to 4 µm or smaller when the inter-column gap S1 of the initial columnar structure 24 is between 1 µm and 5 µm. Even when the gap is made to no more than 10 nm, variation in the thickness of the thermally oxidized film is within 10 %. The columnar structure prepared in accordance with the embodiment of the present invention has a two-layer structure of the thermally oxidized film and semiconductor, or only the oxidized film (layer). In addition, the thermally oxidized film formed over the columnar structure is connected to a thermally oxidized film 26 formed on the surface of the silicon substrate.

When the structure prepared in accordance with Embodiments 1 and 2 of the present invention was heat-treated at a temperature of 800 °C, no deformation of the columnar structure was recognized. The structure in accordance with the present invention is considered to have an excellent resistance to heat treatment thanks to its simple constitution, i.e. the oxidized silicon film or a two-layer structure made of the oxidized silicon film and silicon, and its connection with the substrate.

When a structure having the columnar structure prepared in accordance with Embodiments 1 and 2 of the present invention was filled with water and the water flowed through the structure having the columnar structure at a constant flow velocity, no breakage caused by water pressure was recognized on the columnar structure. The structure in accordance with the present invention is proved to have sufficient column strength for hydrodynamic applications. This is because the thermally oxidized film formed over the structure is connected to that formed on the surface or inside of the substrate. This is also because the thermally oxidized film and silicon are firmly adhered to each other. In addition, since the thermally oxidized film is hydrophilic, it has an advantage of reducing flow resistance provided when water fluid flows.

### Embodiment 3

### Electrophoresis device for DNA separation

A device using the structure in accordance with the present invention is described below. Fig. 8 shows an example of the structure of an electrophoresis device for DNA separation. In the drawing, reference numeral 27 shows a recessed channel formed in the silicon substrate 11. Reference numeral 28 shows a columnar structure with erecting microscopic columns formed in this recessed channel 27. A negative electrode 29 and a positive electrode 30 are provided at the respective end of the channel 27. The channel 27 is filled with a liquid 31. A cover glass 32 is provided over the columnar structure so that the liquid passes only through the columnar structure. The cover glass 32 has an inlet port 33 on the side of the negative electrode 29 and a fluorescent observation port 34 on the side of the positive electrode. Columnar structures having a pitch of columns of 10 nm, 100 nm, 500 nm, 1 µm and 4 µm, respectively, were prepared. The height of the columnar structure was 20 µm.

Next, DNAs were poured from the inlet port 33 and voltage was applied across the positive electrode 29 and the negative electrode 30. The applied voltage was adjusted so as to maintain an electric field of 20V/cm. The DNAs were labeled with fluorescent dye. When the DNAs that had passed through the columnar structure were observed through the observation port 34, the DNAs of different sizes passed through the observation port 34 at different timing. This shows that a columnar structure having microscopic gaps can separate DNAs size by size. In addition, it was proved that a certain DNA to be separated had its optimum inter-column gap and the smaller DNA required the narrower inter-column gap. However, it was also shown that an inter-column gap of 4 µm or smaller could separate any size of DNA. Conventional methods of electrophoresis for DNA separation have used a gel or polymer as the separation carrier and the operation of charging a gel or polymer was one of factors that had prolonged analysis time. The separation device in accordance with the present invention does not require charging the separation carrier. The device has another advantage of that the optimum design and preparation of the separation carrier is possible, so that separation capability is improved. Moreover, since the separation device is prepared on a silicon substrate, a DNA amplifying device and a detecting device are integrally prepared on a single silicon chip. This constitution can reduce analysis time and manufacturing cost.

DNAs are driven using electric field (electrophoresis) in Embodiment 3. The same effects can be expected by using force from a fluid.

## Claims

1. A structure for use in DNA separation, including:
- a semiconductor substrate (11); and
- a plurality of columns (12) formed on the semiconductor substrate (11) and of which at least the surface layer is made of an oxide of the semiconductor,
- wherein passage of DNAs through the columns (12) enables separation of the DNAs.

2. The structure according to claim 1,
wherein the height of the columns (12) from a surface of the semiconductor substrate (11) is between 1 µm and 1 mm.

3. The structure according to claim 1 or 2,
wherein part of the column (12) is embedded in a hole (15) provided in a surface of the semiconductor substrate (11).

4. The structure according to any of claims 1 to 3,
wherein the pitch of the adjacent columns (12) is between 10 nm and 4 µm.

5. A method of manufacturing a structure for use in DNA separation, including:
- a step of preparing a semiconductor substrate (11);
- a step of forming a mask layer (13) with a plurality of openings on the surface of the semiconductor substrate (11);
- an etching step of immersing the semiconductor substrate (11) in an etching liquid so as to etch the semiconductor substrate (11) exposed in the openings and to form holes (15);
- a step of thermally oxidizing the semiconductor substrate (11) and forming a thermally oxidized film (16) so that the film (16) covers the surface of the holes (15);
- a step of removing the mask layer (13); and
- a step of etching the semiconductor substrate (11) from the surface thereof so that the thermally oxidized film (16) protrudes from the surface of the semiconductor substrate (11) and forming columns (12) of which at least the surface is made of the thermally oxidized film (16).

6. The method according to claim 5,
wherein the etching step is an electrolytic etching step in which the semiconductor substrate (11) is immersed in a solution containing hydrofluoric acid (19) and used as an anode for etching.

7. A DNA separation device for use in DNA separation, including:
- a semiconductor substrate (11a);
- a recess (27) provided in the surface of the semiconductor substrate (11a) so as to hold a liquid (31);
- a plurality of columns (28) provided at the bottom of the recess (27) of which at least the surface layer is made of an oxide of the semiconductor; and
- a pair of electrodes (29, 30) sandwiching the columns (28),
- wherein a voltage is applied across the electrodes (29, 30) so that DNAs in the liquid (31) held in the recess (27) perform electrophoresis through the columns (28).

8. The device according to claim 7,
wherein the height of the columns (28) from the bottom of the recess (27) is between 1 µm and 1 mm.

9. The device according to claim 7 or 8,
wherein the pitch of the adjacent columns (28) is between 10 nm and 4 µm.
